(19) Europäisches Patentamt
European Patent Office
Office européen des brevets

(11) Publication number: **0 088 642**
**A2**

(12) **EUROPEAN PATENT APPLICATION**

(21) Application number: **83301321.2**

(22) Date of filing: **10.03.83**

(51) Int. Cl.³: **C 07 D 491/22**
//
A61K31/47 ,(C07D491/22, 311/00, 221/00, 221/00, 209/00)

(30) Priority: **10.03.82 JP 36344/82**

(43) Date of publication of application: **14.09.83**
**Bulletin 83/37**

(84) Designated Contracting States: **BE CH DE FR GB IT LI NL SE**

(71) Applicant: **KABUSHIKI KAISHA YAKULT HONSHA, 1-19, Higashishinbashi 1-chome, Minato-ku Tokyo 105 (JP)**

(72) Inventor: **Miyasaka, Tadashi, 27-11, Aobadai 1-chome Midori-ku, Yokohama-shi Kanagawa-ken (JP)**
Inventor: **Sawada, Seigo, 26-12, Okusawa 5-chome, Setagaya-ku Tokyo (JP)**
Inventor: **Nokata, Kenichiro, 7-24, Sakuracho 2-chome, Koganei-shi Tokyo (JP)**
Inventor: **Mutai, Masahiko, 988, Shimizu 4-chome, Higashiyamato-shi Tokyo (JP)**

(74) Representative: **Allard, Susan Joyce et al, BOULT, WADE & TENNANT 27 Furnival street, London EC4A 1PQ (GB)**

(54) **New camptothecin derivatives and process for their preparation.**

(57) New camptothecin derivatives of the general formula:

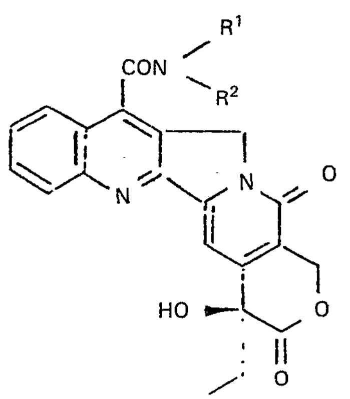

wherein $R^1$ and $R^2$ each stands for a hydrogen atom, a substituted or unsubstituted alkyl group, a substituted or unsubstituted aralkyl group or a substituted or unsubstituted aryl group and $R^1$ and $R^2$, taken together with the nitrogen atom to which they are attached, may form a cyclic group. These new derivatives are useful intermediates for preparing pharmaceuticals or other classes of new camptothecin derivatives. These present new derivatives are prepared by treating camptothecin-7-carboxylic acid first with a carboxyl group-activating reagent and then with ammonia or the corresponding amine.

EP 0 088 642 A2

NEW CAMPTOTHECIN DERIVATIVES AND PROCESS FOR THEIR PREPARATION

This invention relates to new camptothecin derivatives and to processes for the preparation thereof. More particularly, this invention relates to camptothecin-7-carboxamide and its derivatives, which are useful as intermediates in the preparation of pharmaceuticals or other classes of new camptothecin derivatives, as well as to processes for the preparation of these new camptothecin derivatives.

Camptothecin is a cytotoxic alkaloid isolated from the leaves and bark of Camtotheca accuminata (Nyssaceae), a plant native to China, which has a pentacyclic structure consisting of a characteristic fuse 5-ring system of quinoline (rings A and B), pyrroline (ring C), α-pyridone (ring D) and a six-membered lactone (ring E) and is distinguished by displaying a strong inhibitory activity toward the biosynthesis of nucleic acid. In addition, camptothecin is a unique anti-tumour substance characterized by its rapid and reversible action and its lack of

any cross-tolerance with the existing anti-tumour agents and by exhibiting a strong anti-tumour activity against experimentally transplanted carcinoma such as leukemia L-1210 in mice or Walker 256 tumour in rats. Although camptothecin is still regarded as one of the most potent substances possessing anti-tumour activity, the use of this compound itself for clinical treatments is very limited because of its high toxicity.

Accordingly, a number of attempts have been made to reduce the toxicity of camptothecin while maintaining its anti-tumour activity by converting camptothecin chemically into its derivatives. The chemical modifications so far reported are mainly on rings D and/or E of camptothecin, but the results of such modifications revealed only a failure in maintaining the expected anti-tumour activity and a poor improvement in toxicity [J. Med. Chem., 19 (1976), 675]. From the chemotherapeutic point of view, therefore, it is of importance that the chemical modifications of camptothecin should be restricted to rings A, B and C without effecting any change to rings D and E which are considered to be one of the essential structural elements for maintaining the characteristic biological activities.

Except for a method for functionalizing the 12-position of camptothecin reported in 1976, which comprises

a series of troublesome conversion and purification operations starting with nitration at the 12-position [P. Pei-chuang et al., Hau Hsueh Hsueh Pao 33 (1975), 71; Chem. Abstr. 84 (1976) 115629p], no success was reported until 1979 in connection with the chemical functionalization of camptothecin in a moiety involving rings A, B and C. This is probably ascribable to the reasons that camptothecin itself is only sparingly soluble in various organic solvents and that camptothecin which possesses heterocyclic rings in its molecule is resistant to electrophilic reactions conventionally carried out on aromatic rings.

We have developed processes for introducing (1) a hydroxymethyl group into the 7-position, (2) a hydroxy group into the 5-position and (3) an alkyl or aralkyl group into the 7-position of camptothecin efficiently in a single step, and prepared a great number of new camptothecin derivatives possessing anti-tumour activity with slight toxicity from the 5- and 7-substituted camptothecin obtained according to the above processes (Japanese Laid-open Patent Applns. Nos. Sho. 56-12391, 56-12392, 56-19293, 56-12394, 56-158786, 57-116075 and 57-116076; USSN 166,953 and 336,494; and DOS 30 26 172). Furthermore, we have developed a two-step process for preparing camptothecin -7-carboxylic acid in an efficient manner.

However, the types of camptothecin derivatives prepared according to these processes are still limited.

There is still a great demand for further research on the relation between the substituents in camptothecin derivatives and anti-tumour activity and/or toxicity, and for developing further new classes of camptothecin derivatives possessing a low toxicity.

We have now developed new camptothecin derivatives which are useful as intermediates for the preparation of pharmaceuticals or further new classes of camptothecin derivatives and processes for the preparation of such new camptothecin derivatives.

In accordance with the present invention, there are provided new camptothecin derivatives of the general formula:

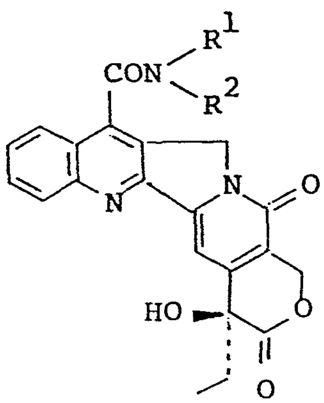

(I)

wherein $R^1$ and $R^2$ each independently represent a hydrogen atom, a substituted or unsubstituted alkyl group, a substituted or unsubstituted aralkyl group or a substituted

or unsubstituted aryl group or $R^1$ and $R^2$ when taken together with the nitrogen atom to which they are attached, may form a heterocyclic group containing one or two nitrogen, oxygen and/or sulfur atoms.

Furthermore in accordance with the present invention, there is provided a process for the preparation of the camptothecin-7-carboxamides of the general formula (I), which comprises treating camptothecin-7-carboxylic acid first with a carboxyl group-activating reagent to activate the carboxyl group and then with ammonia or a primary or secondary amine of the general formula:

$$HNR^1R^2 \qquad (II)$$

wherein $R^1$ and $R^2$ are as defined above.

The camptothecin-7-carboxamides of the present invention are of importance in developing useful pharmaceuticals and further new classes of camptothecin derivatives.

Reference will now be made in more detail to the process for the preparation of the camptothecin-7-carboxamides of the present invention.

Camptothecin-7-carboxylic acid is dissolved or suspended in an inert organic solvent, i.e. an organic solvent which is generally inert to the reagents used to activate the carboxyl group in a carboxylic acid as well as to activated carboxylic acid derivatives resulting from such activation. Examples of these inert organic solvents

include dimethylformamide (DMF), dimethyl sulfoxide (DMSO), pyridine, methylene chloride, chloroform, dichloroethane, dioxane, ether, tetrahydrofuran, glyme, diglyme, benzene, toluene and acetonitrile. To this solution or suspension is added a carboxyl group-activating reagent, which is generally used in peptide synthesis. Examples of such reagents include N,N'-dicyclohexylcarbodiimide (DCC), N,N'-carbonyldiimidazole, N,N'-diisopropylcarbodiimide, diphenyl chlorophosphate, 2,2'-dipyridyl disulfide, N,N'-di-p-tolycarbodiimide, 1-hydroxybenztriazole, N-hydroxysuccinimide, imidazole, triazole, pyrazole, a chloroformic acid ester, phosphorus oxychloride, pivaloyl chloride and a Vilsmeier reagent to be prepared freshly, for example, from DMF and $POCl_3$.

This activation reaction is generally carried out at a temperature in the range of from -18°C to room temperature. Preferably, the carboxyl group-activating reagent is used in an amount of from 1 to 10 times the equivalent. Preferred results can often be obtained when the reaction is carried out with triethylamine added in an amount of from 0.1 to 10 times the equivalent. The reaction mixture is generally stirred for 5 to 30 minutes to form the activated carboxylic acid. To the resulting reaction mixture is added ammonia or a primary or secondary amine of the general formula (II) in an amount of from 1 to 50

times the equivalent. Where the ammonia or amine is used in the form of a salt such as the hydrochloride or sulfate, it is preferred to add triethylamine to the reaction system in an equimolar amount or in a small excess. The camptothecin-7-carboxamides corresponding to the ammonia or primary or secondary amine can be obtained by working up the reaction mixture. Working up of the reaction mixture can be carried out in general in the following manner.

The reaction mixture is stirred for a period of from 0.5 to 12 hours, under cooling with ice or at room temperature. The solvent is evaporated under reduced pressure and the residue is washed with water or subjected to column chromatography on silica gel.

Examples of the primary amine mentioned above include substituted or unsubstituted lower alkylamines such as methylamine, ethylamine, propylamine, butylamine, ethanolamine, N,N'-dimethylethylenediamine, 3-diethylaminopropylamine and cyclic amines (e.g. cyclohexylamine, cyclopentylamine and cycloheptylamine); aralkylamines such as substituted or unsubstituted benzylamine; arylamines such as substituted or unsubstituted aniline and pyridylamine; and amino acids and derivatives thereof such as glycine, glycine ethyl ester, alanine, phenylalanine, leucine, tyrosine, serine and methionine. Examples of the

secondary amine mentioned above include dialkylamines such as dimethylamine, diethylamine and dipropylamine. Examples of the amines of the general formula (II) wherein $R^1$ and $R^2$, taken together with the nitrogen atom to which they are attached, form a cyclic group include piperidine, pyrrolidine, N-methylpiperazine, morpholine and the like heterocyclic amines.

The preparation of camptothecin-7-carboxylic acid as the above starting material is referred to UK Patent Application GB2056973.

The present invention will now be illustrated in more detail by way of Examples.

Example 1 (Preparation of camptothecin-7-carboxamide)

Camptothecin-7-carboxylic acid (50 mg, 0.128 mmol) is suspended in methylene chloride (10 ml). To this suspension are added, with stirring at about -18°C, triethylamine (80μl, 0.572 mmol) and ethyl chloroformate (80 μl, 0.843 mmol). The resultant mixture is stirred at about -18°C for 0.5 hour, and gaseous ammonia is blown thereinto over a period of 10 minutes. The reaction mixture is further agitated at room temperature for 3 hours. The solvent is then distilled off under reduced pressure and water (50 ml) is added to the residue. The precipitated crystals are collected by filtration whereby 45 mg (yield: 90.2%) of the title compound is obtained.

M.P. 293-295°C (dec.)(from $CH_3OH$ - $H_2O$ - pyridine)

$IR\nu_{max}^{KBr}cm^{-1}$: 3500, 3330, 3170, 1740, 1675, 1660, 1590,

1382, 1233, 1168, 1050, 780

$^1$H-NMR (in DMSO-$d_6$)δppm: 0.90(3H, t, J=7Hz), 1.88 (2H, q, J=7Hz), 5.32 (2H, s), 5.44 (2H, s), 6.52 (1H, s), 7.37 (1H, s), 7.74-8.34 (6H, m)

MS m/e : 391[$M^+$] ($C_{21}H_{17}N_3O_5$=391)

The above starting material, camptothecin-7-carboxylic acid, is prepared as mentioned in UK Patent Application GB2056973.

Example 2 (Preparation of camptothecin-7-N-ethylcarboxamide)

Camptothecin-7-carboxylic acid (50 mg, 0.128 mmol) is suspended in methylene chloride (10 ml). To this suspension are added, with stirring at about -18°C, triethylamine (40 μl, 0.286 mmol) and ethyl chloroformate (30 μl, 0.316 mmol). The resultant mixture is stirred at about -18°C for 0.5 hour. To the mixture is added ethylamine (500 μl) dehydrated with anhydrous sodium carbonate. Methylene chloride (40 ml) is added to the resultant reaction mixture and the mixture is washed with a 10% aqueous solution of acetic acid. The methylene chloride layer is dried over anhydrous magnesium sulfate and evaporated until dryness under reduced pressure. The residue is purified by column chromatography on silica gel, whereby 35 mg (yield: 65.5%) of the title compound is obtained. M.P. 263-266°C (from $C_2H_5OH$ - $H_2O$)

IR$\nu_{max}^{KBr}$cm$^{-1}$: 3230, 2960, 1760, 1640, 1580, 1225, 1155,

770, 722

$^1$H-NMR (in DMSO-$d_6$)δppm: 0.89 (3H, t, J=7Hz), 1.25 (3H, t, J=7Hz), 1.87 (2H, q, J=7Hz), 3.30 (2H, m), 5.28(2H, s), 5.43 (2H, s), 6.52 (1H, s), 7.37 (1H, s), 7.83-8.26 (4H, m), 8.96 (1H, m)

MS m/e : 419 [$M^+$] ($C_{23}H_{21}N_3O_5$=419)

Example 3 (Preparation of camptothecin-7-N-cyclohexyl-carboxamide)

Camptothecin-7-carboxylic acid (100 mg, 0.255 mmol) is suspended in dioxane (50 ml). To this suspension is added DCC (100 mg, 0.485 mmol) at room temperature and the mixture is stirred for 0.5 hour. To this mixture is added cyclohexylamine (2 ml) and the resultant mixture is stirred at room temperature for 6 hours. The solvent is then distilled off under reduced pressure and the residue is purified by column chromatography on silica gel ($CHCl_3$ as eluent), whereby 35 mg (yielf: 29.0%) of the title compound is obtained. M.P. 252-254°C (from $C_2H_5OH$ - $H_2O$)

IR$\nu^{KBr}_{max}$$cm^{-1}$: 3260, 2930, 1755, 1660, 1595, 1550, 1222, 1158, 775, 720

$^1$H-NMR (in DMSO-$d_6$)δppm: 0.89 (3H, t, J-7Hz), 1.3-1.9 (12H, m), 3.90 (1H, br.), 5.24 (2H, s), 5.42 (2H, s), 6.49 (1H, s), 7.37 (1H, s),

7.73-8.24 (4H, m), 8.78 (1H, d, J=8.5Hz)

MS m/e : 473 $[M^+]$ ($C_{27}H_{27}N_3O_5$=473)

Example 4 (Preparation of camptothecin-7-N-benzylcarboxamide)

A mixture of DMF (1 ml) and $POCl_3$ (1 ml) is heated at 50°C for 30 minutes to prepare a Vilsmeier reagent. To the thus prepared reagent is added camptothecin-7-carboxylic acid (50 mg, 0.128 mmol) and the mixture is stirred at about -18°C for 30 minutes. After addition of benzylamine (1 ml), the mixture is stirred at room temperature for 30 minutes. The reaction mixture is evaporated until dryness under reduced pressure and water (20 ml) is added to the residue. The prepcipitated crystals are collected by filtration, whereby 48 mg (yield: 78.0%) of the title compound is obtained.

M.P. 267-269°C (from $CH_3OH$ - $H_2O$)

$IR\nu_{max}^{KBr}cm^{-1}$: 3540, 3260, 2940, 1740, 1655, 1590, 1550 1158, 1042, 725, 702

$^1$H-NMR (in DMSO-$d_6$)δppm: 0.89 (3H, t, J=7Hz), 1.88 (2H, q, J=7Hz), 4.67 (2H, d, J=5.8Hz), 5.28 (2H, s), 5.43 (2H, s), 6.52 (1H, s), 7.30-7.50 (6H, m), 7.75-8.28 (4H, m), 9.42 (1H, t, J=5.8 Hz)

MS m/e : 481 $[M^+]$ ($C_{28}H_{23}N_3O_5$=481)

Example 5 (Preparation of ethyl 2-(camptothecin-7-carboxamido)-acetate)

Camptothecin-7-carboxylic acid (50 mg, 0.128 mmol) is suspended in methylene chloride (10 ml). To this suspension are added, at about -18°C, triethylamine (80 μl, 0.572 mmol) and ethyl chloroformate (80 μl, 0.843 mmol), and the mixture is stirred for 0.5 hour. To the mixture are added, at about -18°C, glycine ethyl ester hydrochloride (50 mg, 0.358 mmol) and triethylamine (40μℓ) and the resultant mixture is stirred at room temperature for 3 hours. After addition of methylene chloride (50 ml), the mixture is washed with a 10% aqueous solution of acetic acid. The methylene chloride layer is dried over anhydrous magnesium sulfate and evaporated under reduced pressure until dryness. The residue is purified by column chromatography on silica gel ($CHCl_3$ as eluent), whereby 22 mg (yield: 36.0%) of the title compound is obtained. M.P. 269-272°C (from $C_2H_5OH - H_2O$)

$IR\nu_{max}^{KBr}cm^{-1}$: 3300, 3230, 2970, 1753, 1657, 1589, 1200, 1160, 1055, 775, 722

$^1$H-NMR (in $CDCl_3$) δppm: 0.96 (3H, t, J=7.3Hz), 1.42 (3H, t, J=7.3 Hz), 1.77 (2H, q, J=7.3 Hz), 4.38 (2H, q, J=7.3 Hz), 4.45 (2H, d, J=7Hz), 5.09 (2H, ABq), 5.16 (2H, s), 7.49 (1H, s), 7.55-8.27 (5H, m)

MS m/e : 477 [$M^+$] ($C_{25}H_{23}N_3O_7$=477)

Example 6 (Preparation of camptothecin-7-N-phenylcarboxamide)

Camptothecin-7-carboxylic acid (50 mg, 0.128 mmol) is suspended in methylene chloride (10 ml). To this suspension

are added, with stirring at about -18°C, triethylamine (80 µl, 0.572 mmol) and ethyl chloroformate (80 µl, 0.843 mmol), and the suspension is stirred at about -18°C for 0.5 hour. Aniline (2 ml) is added to this suspension and the resultant mixture is stirred at room temperature for 12 hours. After the reaction, the solvent is evaporated off under reduced pressure and the residue is shaked with methylene chloride (100 ml) and a 10% aqueous solution of acetic acid (50 ml). The methylene chloride layer is dried over anhydrous magnesium sulfate and evaporated under reduced pressure until dryness. The residue is purified by column chromatohraphy on silica gel ($CHCl_3$ as eluent), whereby 15 mg (yield: 25%) of the title compound is obtained. M.P. 255-257°C (from $CH_3OH$ - $H_2O$)

$IR\nu_{max}^{KBr}cm^{-1}$: 3300, 2940, 1740, 1655, 1600, 1190, 1040, 720

MS m/e : 467[$M^+$] ($C_{27}H_{21}N_3O_5$=467)

Example 7 (Preparation of N,N-dimethylcamptothecin-7-carboxamide)

Camptothecin-7-carboxylic acid (50 mg, 0.128 mmol) is dissolved in DMF (10 ml). To this solution are added, with stirring at about -18°C, triethylamine (80 µl, 0.572 mmol) and ethyl chloroformate (80 µl, 0.843 mmol) and the mixture is stirred at about -18°C for 0.5 hour. Dimethylamine (2 ml) is added to the mixture at about -18°C and the resultant mixture is stirred

at room temperature for 0.5 hour. After addition of acetic acid (2 ml), the mixture is stirred for 0.5 hour. The solvent is then distilled off under reduced pressure and the residue is purified by column chromatography on silica gel ($CHCl_3$ as eluent), whereby 48 mg (yield: 89.6%) of the title compound is obtained. M.P. 283-285°C (from $CH_3OH$ - $H_2O$)

$IR\nu_{max}^{KBr}$ $cm^{-1}$: 3420, 2930, 1748, 1658, 1630, 1600, 1228, 1152, 1048, 772, 722

$^1H$-NMR (in DMSO-$d_6$) δppm: 0.89 (3H, t, J=7Hz), 1.88 (2H, q, J=7Hz), 2.80 (3H, s), 3.21 (3H, s), 5.15 (2H, ABq), 5.43 (2H, s), 6.53 (1H, s), 7.37 (1H, s), 7.75-8.28 (4H, m)

MS m/e : 419 [$M^+$] ($C_{23}H_{21}N_3O_5$=419)

Example 8 (Preparation of N,N'-diethylcamptothecin-7-carboxamide)

Camptothecin-7-carboxylic acid (50 mg, 0.128 mmol) is suspended in methylene chloride (10 ml). To this suspension are added, with stirring at about -18°C, triethylamine (80 μl, 0.572 mmol) and ethyl chloroformate (80 μl, 0.843 mmol), and the mixture is stirred at about -18°C for 0.5 hour. Diethylamine (2 ml) is added to this mixture at about -18°C and the resultant mixture is stirred at room temperature for 0.5 hour. After addition of acetic acid (2 ml), the mixture is stirred for 0.5 hour and the solvent is evaporated off under reduced pressure. The residue is purified by column chromatography

on silica gel ($CHCl_3$ as eluent), whereby 20 mg (yield: 35.0%) of the title compound is obtained. M.P. 261-264°C (from $C_2H_5OH$ - $H_2O$)

$IR\nu_{max}^{KBr}cm^{-1}$: 3400, 2960, 2925, 1754, 1658, 1635, 1591, 1458, 1217, 1156, 1052, 780, 762, 722

MS m/e : 447 [$M^+$] ($C_{25}H_{25}N_3O_5$=447)

Example 9 (Preparation of 4-(camptothecin-7-carbonyl)morpholine)

Camptothecin-7-carboxylic acid (50 mg, 0.128 mmol) is suspended in methylene chloride (10 ml). To this suspension are added, with stirring at about -18°C, triethylamine (80 µl, 0.572 mmol) and ethyl chloroformate (80 µl, 0.843 mmol), and the mixture is stirred at about -18°C for 0.5 hour. Morpholine (2 ml) is added to this mixture at about -18°C and the resultant mixture is stirred at room temperature for 0.5 hour. After addition of acetic acid (2 ml), the mixture is stirred for 5 hours, and the solvent is evaporated off under reduced pressure. The residue is purified by column chromatography on silica gel ($CHCl_3$ as eluent), whereby 28 mg (yield: 47.5%) of the title compound is obtained.

$^1H$-NMR (in $CDCl_3$) δppm: 1.01 (3H, t, J=7Hz), 1.93 (2H, q, J=7Hz), 3.55 (4H, t, J=8Hz), 3.94 (4H, br.t), 5.25 (2H, s), 5.46 (2H, ABq), 7.66-8.29 (5H, m)

MS m/e : 461 [$M^+$] ($C_{25}H_{23}N_3O_6$=461)

Example 10 (Preparation of Ethyl 2-(camptothecin-7-carboxamido) acetate)

Camptothecin-7-carboxylic acid (100 mg, 0.255 mmol) is dissolved in pyridine (5 ml) and glycine ethyl ester (130 mg, 1.27 mmol) is added to the solution. To the mixture is added dropwise $POCl_3$ (50 µl, 0.55 mmol) under cooling with ice. The resultant mixture is stirred for 30 minutes and the solvent is evaporated off under reduced pressure. The residue is dissolved in chloroform and the solution is washed with water. The chloroform layer is dried over anhydrous magnesium sulfate and evaporated under reduced pressure until dryness. The residue is purified by column chromatography on silica gel (chloroform as eluent), whereby 45 mg (yield: 37.0%) of the title compound is obtained.

M.P. 269-272°C (from $C_2H_5OH-H_2O$)

$IR\nu_{max}^{KBr}cm^{-1}$: 3300, 3230, 2970, 1753, 1657, 1589, 1200, 1160, 1055, 775, 722

$^1H$-NMR (in $CDCl_3$) δppm: 0.96(3H, t, J=7.3Hz), 1.42(3H, t, J=7.3Hz), 1.77(2H, q, J=7.3Hz), 4.38(2H, q, J=7.3Hz), 4.45(2H, d, J=7Hz), 5.09(2H, ABq), 5.16(2H, s), 7.49(1H, s), 7.55-8.27(5H, m)

MS m/e: 477 [$M^+$], for $C_{25}H_{23}N_3O_7$=477

Example 11 (Preparation of Ethyl 2-(camptothecin-7-carboxamido) propionate)

The reaction and work-up is carried out in the same manner as in Example 10, except that alanine ethyl ester (130 mg, 1.11 mmol) is used in stead of glycine ethyl ester, whereby 95 mg (yield: 76%) of the title compound is obtained.

M.P. ca. 235-238.5°C (dec.)

$^1$H-NMR (in DMSO-$d_6$) δppm: 0.89(3H, t, J=7Hz), 1.32(3H, t, J=7Hz), 1.46(3H, d, J=7Hz), 1.89(2H, q, J=7Hz), 4.25(2H, q, J=7Hz), 4.67(1H, m), 5.31(2H, s), 5.44(2H, s), 6.53 (1H, s), 7.38(1H, s), 7.77-8.01(2H, m), 8.16-8.28 (2H, m), 9.38(1H, d, J=7Hz)

Example 12 (Preparation of Ethyl 2-(camptothecin-7-carboxamido)-3-phenylpropionate)

The reaction and work-up is carried out in the same manner as in Example 10, except that phenylalanine ethyl ester (150 mg, 0.776 mmol) is used in stead of glycine ethyl ester, whereby 130 mg (yield: 90.2%) of the title compound is obtained.

M.P. ca. 251.5-253.5°C (dec.)

$^1$H-NMR (in DMSO-$d_6$) δppm: 0.89(3H, t, J=7Hz), 1.32(3H, t, J=7Hz), 1.89(2H, q, J=7Hz), 2.87-3.39(2H, m), 4.27(2H, q, J=7Hz), 5.00(1H, m), 5.08(2H, s), 5.45(2H, s), 6.52 (1H, s), 7.34(5H, s), 7.68(1H, s), 7.30-8.20(4H, m), 9.41(1H, d, J=8Hz)

Example 13 (Preparation of N-(N',N'-diethylaminopropyl)-camptothecin-7-carboxamide)

The reaction is carried out in the same manner as in Example 10, except that N,N-diethyltrimethylenediamine (43 mg, 0.33 mmol) is used in stead of glycine ethyl ester and that the amount of $POCl_3$ used is 30 μl (0.33 mmol). The reaction mixture is concentrated under reduced pressure until dryness and the residue is recrystallized from methanol-acetone, whereby 50 mg (yield: 36.2%) of the title compound is obtained.

M.P. ca. 248.5-251°C (dec.)

$^1$H-NMR (in DMSO-$d_6$) δppm: 0.89(3H, t, J=7Hz), 1.23(6H, t, J=7Hz), 1.80-2.10(4H, m), 3.10-3.60(8H, m), 5.37(2H, s), 5.43 (2H, s), 6.53(1H, s), 7.38(1H, s), 7.65-8,27(4H, m), 9.27(1H, t, J=5.5Hz), 10.36(1H, br. s)

Example 14 (Preparation of N-(N',N'-diethylaminoethyl) camptothecin-7-carboxamide hydrochloride)

The reaction is carried out in the same manner as in Example 10, except that N,N-diethylethylenediamine (38 mg, 0.33 mmol) is used in stead of glycine ethyl ester and that the amount of $POCl_3$ used is 30 μl (0.33 mmol). The reaction mixture is concentrated under reduced pressure until dryness. The residue is recrystallized from methanol-acetone, whereby 80 mg (yield: 59.7%) of the title compound is obtained.

M.P. ca. 230-235°C (dec.)

$^1$H-NMR (in DMSO-$d_6$) δppm: 0.91(3H, t, J=7Hz), 1.25(6H, t, J=7Hz), 1.88(2H, q, J=7Hz), 3.00-3.60(8H, m), 5.28(2H, s), 5.39 (2H, s), 6.51(1H, s), 7.30(1H, s), 7.43-8.01(4H, m),

8.80(1H, d, J=8Hz), 9.30(1H, s)

Example 15 (Preparation of 1-(camptothecin-7-carbonyl)-4-methylpiperazine hydrochloride)

The reaction is carried out in the same manner as in Example 10, except that N-methylpiperazine (30 mg, 0.33 mmol) is used in stead of glycine ethyl ester and that the amount of $POCl_3$ used is 30 μl (0.33 mmol). The reaction mixture is concentrated under reduced pressure until dryness. The residue is recrystallized from methanol, whereby 65 mg (yield: 50%) of the title compound is obtained.

M.P. ca. 250-254°C (dec.)

Example 16 (Preparation of Ethyl 6-(camptothecin-7-carboxamido)-n-caproate)

The reaction and work-up is carried out in the same manner as in Example 10, except that ethyl 6-amino-n-caproate (160 mg, 1.00 m mol), whereby 82 mg (yield: 60.3%) of the title compound is obtained.

M.P. 253-256°C (dec.)

$^1$H-NMR (in DMSO-$d_6$)δppm: 0.97(3H, t, J=7Hz), 1.18(3H, t, J=7Hz), 1.20-1.50(6H, m), 1.88(2H, q, J=7Hz), 2.26(2H, t, J=7Hz), 2.74(2H, m), 4.04(2H, q, J=7Hz), 5.27(2H, s), 5.44(2H, s), 6.54(1H, s), 7.37(1H, s), 7.50-8.30 (4H, m), 8.92(1H, br, t)

Example 17 (Preparation of Ethyl 2-(camptothecin-7-carboxamido)-n-caproate)

The reaction and work-up is carried out in the same manner as in Example 10, except that leucine ethyl ester (160 mg, 1.00 mmol) is used instead of glycine ethyl ester, whereby 88 mg (yield: 64.2%) of the title compound is obtained.

M.P. 250-253°C (dec.)

Example 18; 2-(Camptothecin-7-carboxamido)acetic Acid and Its Mono Sodium Salt.

Ethyl 2-(camptothecin-7-carboxamido)acetate (30 mg, 0.063 mmol) obtained as described in Ex. 10 was suspended in 0.1 N NaOH aq. solution (1.9 ml) and the mixture was stirred vigorously for 8 hr. at room temperature. The alkaline solution was neutralized with 0.1 N HCl aq. solution (1.9 ml) and the resulting precipitated material was collected by filtration. The title acid was thus obtained in 90% yield (25 mg) after drying in vacuo.

The sodium salt of the acid was obtained in the ordinary manner, for example, the acid was dissolved in water (1 ml) containing equimolar amounts of $NaHCO_3$ and the solution was filtered, and then evaporated to dryness under reduced pressure and/or was lyophilized. The sodium salt was obtained in quantitative yielf as a fine yellow powder.

IR $\nu_{max}^{KBr}$ $cm^{-1}$; 3410, 2940, 1740, 1660, 1598, 1220, 1152, 800.

Example 19 ; 2-(Camptothecin-7-carboxamido)propionic acid and its Mono Sodium salt.

The ethyl ester of the title acid (30 mg, 0.061 mmol) obtained by the method as described in Ex. 11 was hydrolyzed in 0.1 N NaOH aq. solution at room temperature and the resulting mixture was worked up in the same matter as in Ex. 18. The acid was obtained in a 93% yield as a yellow powder.

The sodium salt of the acid was prepared by the method described in Ex. 18 (lyophilization).

$IR\nu_{max}^{KBr}cm^{-1}$; 3420, 2930, 1745, 1652, 1596, 1230, 1158, 802.

Example 20 ; 2-(Camptothecin-7-carboxamido)-3-phenylpropionic Acid and Its Mono Sodium salt.

The ester of the title acid (30 mg, 0.054 mmol) obtained by the method described in Ex. 12 was hydrolyzed in 0.1 N NaOH aq. solution and the resulting mixture was worked up in the same manner as described in Ex. 18. The acid was obtained in a 95% yield.

The sodium salt of the acid was prepared by the method as described in Ex. 18 (lyophilization).

$IR\nu_{max}^{KBr}cm^{-1}$; 3430, 1736, 1652, 1596, 1225, 1158, 762.

Example 21 ; 6-(Camptothecin-7-carboxamido)-n-caproic Acid and Its Mono Sodium salt.

The ethyl ester of the title acid (30 mg, 0.056 mmol)

obtained by the method described in Ex. 16 was hydrolyzed in 0.1 N NaOH aq. solution at room temperature and the resulting mixture was worked up in the same manner as described in Ex. 18.

The title acid was obtained in an 88% yield.

The sodium salt of the acid was prepared by the method as described in Ex. 18 (lyophilization).

IR$\nu_{max}^{KBr}cm^{-1}$; 3400, 2955, 1740, 1655, 1598, 1226, 1050, 748.

Example 22 ; 2-(Camptothecin-7-carboxamido)-isocaproic Its Sodium Salt.

The ester of the title acid (30 mg, 0.056 mmol) was hydrolyzed in 0.1 N NaOH aq. solution and the resulting mixture was worked up in the same manner as described in Ex. 18. The acid was obtained in any 82% yield.

The sodium salt of the acid was prepared by the method described in Ex. 18 (lyophilization).

IR$\nu_{max}^{KBr}cm^{-1}$; 3400, 2950, 1738, 1652, 1602, 1224, 1154, 1044, 766.

CLAIMS:

1. A camptothecin derivative of the general formula:

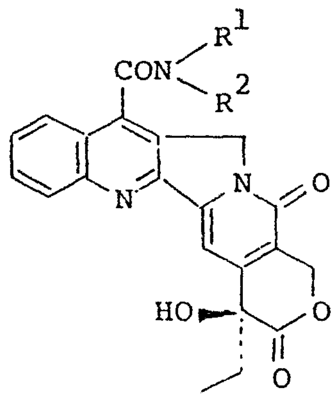

wherein $R^1$ and R: each independently represent a hydrogen atom, a substituted or unsubstituted alkyl group, a substituted or unsubstituted aralkyl group or a substituted or unsubstituted aryl group or $R^1$ and $R^2$, when taken together with the nitrogen atom to which they are attached, may form a heterocyclic group containing one or two nitrogen, oxygen and/or sulfur atoms.

2. Camptothecin-7-carboxamide.

3. Camptothecin-7-N-ethylcarboxamide.

4. Camptothecin-7-N-cyclohexylcarboxamide.

5. Camptothecin-7-N-benxylcarboxamide.

6. Ethyl 2-(camptothecin-7-carboxamido)acetate.

7. Camptothecin-7-N-phenylcarboxamide.

8. N,N-dimethyl-camptothecin-7-carboxamide.

9. N,N-diethyl-camptothecin-7-carboxamide.

10. 4-(camptothecin-7-carbonyl)morpholine.

11. A camptothecin derivative as claimed in claim 1 wherein $R^1$ is a hydrogen atom and $R^2$ is an alkyl or aralkyl group substituted with a carboxyl group.

12. A mono sodium salt of a camptothecin derivative as claimed in claim 11.

13. A camptothecin derivative as claimed in claim 1 wherein $R^1$ is a hydrogen atom and $R^2$ is an alkyl or aralkyl group substituted with an alkoxycarbonyl group.

14. A camptothecin derivative as claimed in claim 1 wherein $R^1$ is a hydrogen atom and $R^2$ is an alkyl or aralkyl group substituted with a dialkylamino group.

15. A hydrochloride of a camptothecin derivative as claimed in claim 14.

16. A process for the preparation of camptothecin derivatives of the general formula:

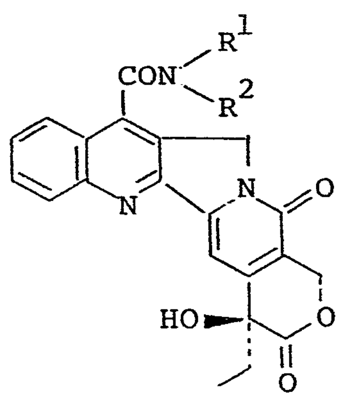

wherein $R^1$ and $R^2$ are each as defined in claim 1 which process comprises treating camptothecin with a carboxyl group-activating reagent and then with ammonia or a primary

or secondary amine of the general formula:

$HNR^1R^2$

wherein $R^1$ and $R^2$ are as defined above.

17. A process as claimed in claim 16 wherein the carboxyly group-activating reagent is N,N'-dicyclohexylcarbodiimide (DCC), N,N'-carbonyldiimidazole, N,N'-diisopropylcarbodiimide, diphenyl chlorophosphate, 2,2'-dipyridyl disulfide, N,N'-di-p-tolycarbodiimide, 1-hydroxybenztriazole, N-hydroxysuccinimide, imidazole, triazole, pyrazol, a chloroformic acid ester, phosphorus oxychloride, pivaloyl chloride or a Vilsmeier reagent.

18. A process according to claim 11, wherein the treatment is carried out in the presence of triethylamine.